**Europäisches Patentamt**

**European Patent Office**

**Office européen des brevets**

(19)

(11) Publication number: **0 097 374**
**A2**

(12) **EUROPEAN PATENT APPLICATION**

(21) Application number: **83106113.0**

(22) Date of filing: **22.06.83**

(51) Int. Cl.³: **A 61 K 9/06**
**A 61 K 47/00**

(30) Priority: **23.06.82 JP 109016/82**

(43) Date of publication of application:
**04.01.84 Bulletin 84/1**

(84) Designated Contracting States:
**BE CH DE FR IT LI NL SE**

(71) Applicant: **SHIONOGI & CO., LTD.**
**12, Dosho-machi 3-chome Higashi-ku**
**Osaka 541(JP)**

(72) Inventor: **Okamoto, Kunio**
**20-78, Sano-dai**
**Izumisano-shi Osaka(JP)**

(72) Inventor: **Nishihara, Yoshitaka**
**47-3, Aoba-dai**
**Izumi-shi Osaka(JP)**

(72) Inventor: **Takehara, Mitsunori**
**12-12, Chiyodadai-cho**
**Kawachinagano-shi Osaka(JP)**

(74) Representative: **Vossius Vossius Tauchner Heunemann**
**Rauh**
**Siebertstrasse 4 P.O. Box 86 07 67**
**D-8000 München 86(DE)**

(54) Corticosteroid topical preparation.

(57) A corticosteroid topical preparation which comprises a monoglyceride of a $C_6$-$C_{10}$ medium-chain fatty acid together with the active ingredient.

EP 0 097 374 A2

This invention relates to a corticosteroid topical preparation for treating inflammation. More particularly, it relates to a new anti-inflammatory preparation in the form of ointment, gel, emulsion, cream and the like. Specifically, it relates to a corticosteroid topical preparation which comprises a monoglyceride of a $C_6$-$C_{10}$ medium-chain fatty acid together with the active ingredient.

In the field of corticosteroid topical preparations it has been an accepted notion that the manifestation of the therapeutic efficacy of the active ingredient mainly depends on such factors as the mixing state of the active ingredient in the base; e.g./dispersing or dissolving, and the proportion, the affinity of the mixed composition to the skin, the absorption efficacy of the active ingredient through the skin and the like. These factors are intricately intertwined and affect the manifestation of the therapeutic efficacy. Therefore, the design of such preparations will hardly depend on simple analyses.

In general, for instance, the active ingredient should be dissolved rather than dispersed in the base to achieve a good manifestation of the efficacy of the active ingredient. Excessive enhancing of the solubility, however, adversely affects the release of the active ingredient from the base and reduces the therapeutic efficacy. Therefore, the base ingredients should be selected so as to suitably arrange the above factors and the proportions should be determined so as to design a preparation with high therapeutic efficacy. Additionally, it is also important to find a new base ingredient for the preparation of a preferred base by a simple procedure.

Under the above circumstances, the inventors have sought to formulate a topical preparation intensifying the efficacy of anti-inflammatory corticosteroids having a high degree of safety and stability and imparting a good feeling when applied to the skin. They finally discovered that a topical preparation containing a monoglyceride of a $C_6$-$C_{10}$ medium-chain fatty acid most efficiently balances the dispersion or solubility of the active ingredient in the base with the release to the skin while also being in contact with other adjuvants. Additionally, they discovered that the preparation remarkably improves skin irritations and shows an excellent physical stability. Thus the present invention was accomplished. Accordingly, the present invention provides a corticosteroid topical preparation for treating inflammation which comprises a monoglyceride of a $C_6$-$C_{10}$ medium-chain fatty acid, especially a saturated fatty acid, together with the active ingredient.

The topical preparation of the present invention contains 0.01 - 2.00 weight percent (abbreviated as wt. % hereinafter) of a corticosteroid and 0.5 - 20 wt. % of monoglyceride of a $C_{6-}C_{10}$ medium-chain fatty acid as indispensable ingredients and may contain one or more of the following pharmacologically acceptable adjuvants.

    a.  1-15 wt. % of a primary, secondary or tertiary $C_2-C_6$ alcohol;

    b.  1-10 wt.% of a polyethylene glycol having a mean molecular weight of about 200-4000;

    c.  1-90 wt.% of an oily substance;

    d.  0.5-15 wt.% of a non-ionic surfactant; and

    e.  water.

Additionally, the preparation may contain suitable amounts of stabilizing agents, solubilizing agents and the like.

Examples of the active ingredient, which is a corticosteroid of the present invention are betamethasone valerate, betamethasone dipropionate, alclometasone dipropionate, hydrocortisone acetate, dexamethasone, prednisolone, diflucortolone valerate, triamcinolone acetonide, fluocinonide, fluocinolone acetonide, beclometasone dipropionate and the like; preferred are betamethasone valerate, betamethasone dipropionate, alclometasone dipropionate, dexamethasone, triamcinolone acetonide, fluocinonide and fluocinolone acetonide; most preferred are betamethasone valerate, betamethasone dipropionate and alclometasone dipropionate. A normally effective amount of the active ingredient as topical drug, namely, about 0.01-2.00 wt. %, preferably 0.05-1.0 wt. %, is contained in the preparation. Some antibiotics may be combined with the corticosteroid as the active ingredient.

Examples of the monoglyceride of the $C_6$-$C_{10}$ medium-chain fatty acid are monoglycerides of $C_6$-$C_{10}$ straight chain fatty acids, especially $C_6$-$C_{10}$ saturated fatty acids, more specifically glycerol monohexanoate, glycerol monooctanoate and glycerol monodecanoate. They are used alone or in combination. The proportion of the monoglyceride in the preparation is about 0.5-20 wt.%, preferably 2-15 wt.%, and depends on the solubility of the active ingredient; A low proportion is sufficient for corticosteroids having a high solubility in the monoglyceride, such as betamethasone valerate, betamethasone dipropionate and the like, but a high proportion is necessary for those having poor solubility, such as hydrocortisone acetate, alclometasone dipropionate and the like. The proportion should also depend on the kind and the amount of the further ingredients.

One kind of various pharmacologically acceptable adjuvants is a $C_2$-$C_6$ primary, secondary or tertiary alcohol, i.e. a          straight or branched alcohol, such as         ethanol, isopropanol, ethylene glycol, propylene glycol, triethylene glycol, 1,3-butanediol, 1,5-pentanediol, 1,6-hexanediol, glycerol, 1,2,6-hexanetriol and the like.  The alcohol is contained in the preparation in a proportion of about 1-15 wt. %. preferably about 5-10 wt. %.

A further possible adjuvant, i.e. a polyethylene glycol/ having a mean molecular weight of about 200 to 4000, may be selected from  polyethylene glycol 200, 300, 400, 600, 1000, 1500, 1540, 4000 and the like. These are employed in a proportion of about 1-10 wt. %, preferably about 2-5 wt. %,

based on the / final preparation.  Polyethylene glycol  weight of the

is usually used in combination with an alcohol as mentioned above since polyethylene glycol alone does not sufficiently-contribute to the efficacy of the active ingredient.

Although the combination is well-known in the field of ointments, the mixture of the polyethylene glycol and the alcohol should not be used in such an excessive proportion as to cause the dissolution of the ac-

tive ingredient,which leads to / so-called syneresis of the  the

ointment.  Addition of the above monoglyceride in a certain proportion prevents the syneresis and improves the stability and efficacy of the preparation.

A further    pharmacologically acceptable ingredient is an oily substance. It is used for the purpose of adjusting the solubility of the active ingredient and the viscosity of the preparation and improving the feeling
of the preparation when applied.
Examples of this further ingredient are liquid higher alcohols (e.g. 2-hexyldecanol and 2-octyldecanol), solid higher alcohols (e.g. cetyl alcohol and stearyl alcohol), higher fatty acids (e.g. palmitic acid and stearic acid), esters (e.g. isopropyl myristate, 2-octyldodecyl myristate, diethyl sebacate and diisopropyl adipate), liquid, semisolid or solid hydrocarbons (e.g. squalane, liquid paraffin, various kinds of paraffins, vaseline, microcrystalline wax and ceresin), waxes (e.g. beeswax, spermaceti and carnauba wax), triglycerides of natural fatty acid (e.g. castor oil and olive oil), synthetic triglyceride, diglycerides, monoglycerides of higher fatty acid and the like.

The oily substance is generally used in a
based on
proportion of about 1-90 wt. %, / the preparation, while

the choice and combination ratio of the oily substances as well as the proportion to the whole preparation weight depend on the kind of corticosteroid to be used, the preparation form and the kind of the monoglyceride of a $C_6$-$C_{10}$ medium-

chain fatty acid to be used and its proportion. The proof the oily substance
portion/is, for example, about 5-90 wt. % for an ointment and       about 1-50 wt. %, preferably 2-35 wt. %,

**0097374**

for an emulsion and a cream.

An additional adjuvant may be a surfactant. Surfactants are useful for forming a desired dosage form
and obtaining fine and homogeneous dispersions, emulsions
                                The surfactant
and miscellaneous preparations.  / may be selected from
the group of non-ionic surfactants causing little skin irritation. Such non-ionic surfactants include -
sorbitan fatty acid esters, sorbitol fatty acid esters,
polyoxyethylene sorbitan fatty acid esters, polyoxyethylene fatty acid esters, polyoxyethylene alkyl ethers,
polyoxyethylene hydrogenated castor oil derivatives,
polyoxyethylene polyoxypropylene alkyl ethers and the like.

Specific examples thereof are  sorbitan mono-
laurate, sorbitan monopalmitate, sorbitan monostearate,
sorbitan sesquistearate, polyoxyethylene sorbitan mono-
laurate, polyoxyethylene mono-palmitate, polyoxyethylene
sorbitan mono-stearate, polyoxyethylene sorbitan tristearate, polyoxyethylene sorbitan mono-oleate, polyoxyethylene sorbitan tri-oleate, polyoxyethylene sorbitol
mono-laurate, polyoxyethylene sorbitol hexa-stearate,
polyoxyethylene sorbitol tetra-oleate, polyoxyethylene
lauryl ester, polyoxyethylene stearyl ester, polyoxyethylene oleyl ester, polyoxyethylene lauryl ether,
polyoxyethylene cetyl ether, polyoxyethylene stearyl
ether, polyoxyethylene oleyl ether, polyoxyethylene hexadecyl ether, propylene glycol mono-stearate, polyoxypropylene polyoxyethylene cetyl ether and the like. The surfactant / may be used alone or in a mixture in a proportion of about 0.5-15 wt. %, preferably about 1-10 wt. %, based on the preparation.

Stabilizing agents, including anti-oxidants, chelating agents, antiseptics, buffer agents and the like also may/be used in a suitable amount if required.

Solubilizing agents (e.g. crotamiton and benzyl alcohol) may also be applied, if necessary.

A practical embodiment of the preparation of the present invention is shown below. Its ingredients are abbreviated as follows:

monoglyceride: a monoglyceride of a $C_6$-$C_{10}$ medium-chain fatty acid;

alcohol: a primary, secondary or teriary $C_2$-$C_6$ alcohol;

polyethylene glycol: a polyethylene glycol having a mean molecular weight of 200-4000;

surfactant: a non-ionic surfactant;

(1) Ointment 1

| Corticosteroid | 0.01-2.00 wt. % |
|---|---|
| Monoglyceride | 1-20 wt. % |
| Surfactant | 0.5-5 wt. % |
| Oily substance | the balance |

(2)  Ointment 2

Corticosteroid                    0.01-2.00 wt. %

Monoglyceride                     1-15 wt. %

Alcohol                           1-15 wt. %

Surfactant                        0.5-5 wt. %

Oily substance                    the balance

(3)  Ointment 3

Corticosteroid                    0.01-2.00 wt. %

Monoglyceride                     1-15 wt. %

Polyethylene glycol               1-10 wt. %

Surfactant                        0.5-5 wt. %

Oily substance                    the balance

(4)  Ointment 4

Corticosteroid                    0.01-2.00 wt. %

Monoglyceride                     1-15 wt. %

Alcohol and polyethylene glycol   1-10 wt. %

Surfactant                        0.5-5 wt. %

Oily substance                    the balance

(5)  Cream 1

Corticosteroid                    0.01-2.00 wt. %

Monoglyceride                     1-20 wt. %

Surfactant                        0.5-15 wt. %

Oily substance                    2-35 wt. %

Water                             the balance

(6)  Cream 2

| Corticosteroid | 0.01-2.00 wt. % |
| Monoglyceride | 1-20 wt. % |
| Alcohol | 1-15 wt. % |
| Surfactant | 0.5-15 wt. % |
| Oily substance | 2-35 wt. % |
| Water | the balance |

(7)  Cream 3

| Corticosteroid | 0.01-2.00 wt. % |
| Monoglyceride | 1-20 wt. % |
| Polyethylene glycol | 1-10 wt. % |
| Surfactant | 0.5-15 wt. % |
| Oily substance | 2-35 wt. % |
| Water | the balance |

(8)  Cream 4

| Corticosteroid | 0.01-2.00 wt. % |
| Monoglyceride | 1-20 wt. % |
| Alcohol and polyethylene glycol | 1-10 wt. % |
| Surfactant | 0.5-15 wt. % |
| Oily substance | 2-35 wt. % |
| Water | the balance |

The above preparations are only some examples and should not limit the scope of the present invention. The above preparations may contain a stabilizing agent, a solubilizing agent and the like as noted above.

The topical preparation of this invention may be prepared by usual methods for preparing topical preparations. Namely, the active ingredient is dissolved in the monoglyceride of a $C_6-C_{10}$ medium-chain fatty acid which may be mixed with the above mentioned alcohols, polyethylene glycol and/or a part of the oily substance as described above. The solution or mixture is subjected to heating, mixing with other ajuvants, dispersing, emulsifying and the like according to the desired dosage form is then and/cooled to room temperature. Furthermore, a required amount of a stabilizing agent may be added, if desired.

The efficacy and safety of the objective preparation were evaluated through a / vasoconstrictor assay and by a skin irritation test, respectively.

Efficacy and safety evaluation:

1. Efficacy by vasoconstrictor assay (Enclosed method)

Each fixed amount of a preparation according to the working examples and references is applied onto a commercially available adhesive test tape. The test tapes are applied to the test site of the forearm and the back of the healthy human subject and kept there for 4 hours. After removal of the test tapes, the blanching (intesity of vasoconstriction) on the test spot where the test tape was applied is observed for further 2 or 4 hours.   The results are shown in Tables 1 and 2.

The figures in the tables are relative values for the blanching intensity of the spot, calculated on the assumption  that the intensity of the spot treated with the corresponding reference preparation is 100 each. The value is proportional to the extent of vasoconstriction.

2.   Safety proof by "closed patch" test (Skin irritation test)

Each fixed amount of the preparation (including the active ingredient, i.e. the corticosteroid) according to the working ing/examples and the references is placed on a commercially available patch (Finn chamber (Trademark), Taisho Seiyaku Co., & Ltd.).   The test tapes are applied to the test site of the back of a healthy human subject and maintained for 48 hours.   The test tapes are removed and the reddish marks of the test spots (the intensity of the irritation to the skin) are observed after 0.5 and 24 hours.   The scores of both observed spots are summed up and the results are shown in Tables 1 and 2.

The figures in the tables represent relative values from the working examples, calculated in that the intensity of the reddish irritation mark of the corresponding reference preparation is assumed to be 100 each.   The value is proportional to the extent of skin irritation and inversely proportional to the degree of safety.

Table 1 (Ointment)

| Test Preparation | | Efficacy (Vasoconstriction) | | Safety |
|---|---|---|---|---|
| Example No. | Reference | 2nd Hour | 4th Hour | (Irritation) |
| 1 - 1 | A | 130 | 120 | 98 |
| 1 - 1 | B | 147 | 129 | 105 |
| 1 - 2 | C | 210 | 180 | 100 |
| 1 - 3 | D | 150 | 175 | 101 |
| 1 - 3 | E | 162 | 160 | 103 |
| 1 - 4 | F | 161 | 218 | 100 |
| 1 - 5 | G | 208 | 140 | 38 |
| 1 - 6 | H | 312 | 206 | 62 |

Table 2 (Cream)

| Test Preparation | | Efficacy (Vasoconstriction) | | Safety |
|---|---|---|---|---|
| Example No. | Reference | 2nd Hour | 4th Hour | (Irritation) |
| 2 - 1 | I | 275 | 255 | 114 |
| | J | 172 | 221 | 58 |
| 2 - 2 | I | 293 | 231 | 46 |
| | K | 185 | 207 | 64 |
| | L | 227 | 214 | 102 |
| 2 - 3 | I | 191 | 168 | 35 |
| 2 - 4 | M | 144 | 121 | 85 |
| | N | 149 | 118 | 101 |
| 2 - 5 | M | 127 | 108 | 40 |
| | N | 152 | 110 | 87 |
| 2 - 6 | O | 161 | 135 | 67 |

As noted in Tables 1 and 2, the preparations of the working examples are superior to the corresponding reference preparations. Thus, the present invention provides the preparations having high efficacy and safety. In other words, the present invention provides a process for improving the activity of a corticosteroid preparation which comprises adding a monoglyceride of a $C_6$-$C_{10}$ medium-chain fatty acid to the topical preparation.

The following examples are intended to illustrate values of the practical embodiments. The/proportions in Tables 3-1, 3-2, 4-1, 4-2 and 4-3 are weight percent values.

Example 1 (Ointment)

Ointments are prepared as follows:

White vaseline is melted at 70-80°C, other ingredients are added thereto, and mixed homogeneously. The resultant mixture is cooled to room temperature to give the preparations in Tables 3-1 and 3-2.

Example 2 (Cream)

Creams are prepared as follows:

All ingredients except for water are mixed and heated, stirred and blended at 70-80°C. A specific amount of purified water is added thereto. The mixture is stirred, emulsified and cooled to room temperature to give the preparations in Tables 4-1, 4-2 and 4-3.

Table 3-1

| Ingredient | Example No. | | | Reference | | | | |
|---|---|---|---|---|---|---|---|---|
| | 1-1 | 1-2 | 1-3 | A | B | C | D | E |
| Betamethasone Dipropionate | 0.064 | | | 0.064 | 0.064 | | | |
| Betamethasone Valerate | | 0.06 | | | | 0.06 | | |
| Alclometasone Dipropionate | | | 0.05 | | | | 0.05 | 0.05 |
| Glycerol Monohexanoate | | 10.0 | | | | | | |
| Glycerol Monooctanoate | | | 10.0 | | | | | |
| Glycerol Monodecanoate | 5.0 | | | | | | | |
| Glycerol Monostearate | | | | | 10.0 | | | 10.0 |
| Sorbitan Monostearate | 2.0 | | | | | | | |
| Sorbiton Tristearate | | | 2.0 | | | | | |
| Propyleneglycol Monostearate | | 2.0 | | | | | | |
| Diisopropyl Adipate | 5.0 | | | | | | | |
| Paraffin Wax | | 2.0 | | | | | | |
| Liquid Paraffin | | | | 10.0 | | 10.0 | 10.0 | |
| White Vaseline | 87.936 | 85.94 | 87.95 | 89.936 | 89.936 | 89.94 | 89.95 | 89.95 |

Table 3-2

| Ingredient | Example No. | | | Reference | | |
|---|---|---|---|---|---|---|
| | 1-4 | 1-5 | 1-6 | F | G | H |
| Bethamethasone Valerate | | | 0.12 | | | 0.12 |
| Alclometasone Dipropionate | | 0.05 | | | 0.05 | |
| Dexamethasone | 0.1 | | | 0.1 | | |
| Glycerol Monohexanoate | 5.0 | | 5.0 | | | |
| Glycerol Monodecanoate | | 10.0 | | | | |
| Propylene Glycol | | 3.0 | 4.0 | | 10.0 | 10.0 |
| Polyethylene Glycol 300 | | | 2.0 | | | 5.0 |
| Polyethylene Glycol 400 | 2.0 | | | | | |
| Sorbitan Sesquistearate | 2.0 | | | | | |
| Sorbitan Monooleate | | 2.0 | | | 2.0 | |
| Polyethylene Glycol Monostearate | | | 2.0 | | | 2.0 |
| Diethyl Sebacate | 3.0 | | | | | |
| White Beeswax | | 3.0 | 3.0 | | 3.0 | 2.0 |
| Plastibase (50W)* | | | | 99.9 | | |
| White Vaseline | 87.9 | 81.95 | 84.88 | | 84.95 | 80.88 |

*(Trademark)   a mixed base of polyethylene with liquid paraffin (Squibb & Sons Co., Ltd.)

Table 4-1

| Ingredient | Example No. | | | Reference | | | |
|---|---|---|---|---|---|---|---|
| | 2-1 | 2-2 | 2-3 | I | J | K | L |
| Alclometasone Dipropionate | 0.05 | 0.05 | 0.05 | 0.05 | 0.05 | 0.05 | 0.05 |
| Glycerol Monohexanoate | 12.0 | | | | | | |
| Glycerol Monooctanoate | | 6.0 | | | | | |
| Glycerol Monodecanoate | | | 14.0 | | | | |
| Glycerol Monostearate | | | | | | | 6.0 |
| Propylene Glycol | | | | 20.0 | | | |
| Polyoxyethylene (20) Cetyl Ether | 5.0 | | | 3.0 | | | |
| Polyoxyethylene (30) Stearyl Ester | | | 6.0 | | | | 5.0 |
| Polyoxyethylene Sorbitan Mono-Stearate | | 5.0 | | | 5.0 | 5.0 | |
| Crotamiton | | | 1.8 | | | | |
| Diisopropyl Adipate | 5.0 | | 4.0 | | 10.0 | | |
| Diethyl sebacate | | 10.0 | | | | 10.0 | |
| Cetyl Alcohol | 10.0 | 8.0 | 10.0 | 8.0 | 10.0 | 10.0 | 8.0 |
| Liquid Paraffin | | | | 5.0 | | | 10.0 |
| White Beeswax | 5.0 | | | 5.0 | 5.0 | 5.0 | |
| White Vaseline | | 10.0 | 10.0 | 7.0 | | | 10.0 |
| Purified Water | 63.0 | 61.0 | 54.0 | 52.0 | 70.0 | 70.0 | 60.95 |

Table 4-2

| Ingredient | Example No. | | Reference | |
|---|---|---|---|---|
| | 2-4 | 2-5 | M | N |
| Betamethasone Dipropionate | 0.064 | 0.064 | 0.064 | 0.064 |
| Glycerol Monooctanoate | 3.5 | 2.5 | | |
| Glycerol Monodecanoate | 1.5 | | | |
| Glycerol Monostearate | | | | 5.0 |
| Polyoxyethylene (20) Cetyl Ether | 4.0 | | 2.0 | 4.0 |
| Sorbitan Monooleate | | 0.5 | | |
| Polyoxyethylene Sorbitan Mono-oleate | | 1.5 | | |
| 2-Octyldodecanol | | 3.0 | | |
| 2-Octyldodecyl Myristate | 10.0 | | | 10.0 |
| Cetyl Alcohol | 7.0 | 2.0 | 8.0 | 7.0 |
| Liquid Paraffin | | | 6.0 | |
| White Vaseline | 10.0 | | 15.0 | 10.0 |
| Purified Water | 64.0 | 90.0 | 69.0 | 63.936 |

Table 4-3

| Ingredient | Example No. | | | Reference |
|---|---|---|---|---|
| | 2-6 | 2-7 | 2-8 | 0 |
| Betamethasone Valerate | 0.12 | | | 0.12 |
| Dexamethasone | | 0.1 | | |
| Prednisolone | | | 1.0 | |
| Glycerol Monooctanoate | | 4.0 | 16.0 | |
| Glycerol Monodecanoate | 8.0 | 1.0 | | |
| Polyoxyethylene (20) Cetyl Ether | | 4.0 | | 2.0 |
| Polyoxyethylene (30) Stearyl Ester | 4.5 | | | |
| Sorbitan Sesquistearate | 1.0 | | | |
| Polyoxyethylene Sorbitan Mono-stearate | | | 5.0 | |
| Crotamiton | | | 1.8 | |
| Diethyl Sebacate | | 2.0 | | |
| 2-Octyldodecyl Myristate | | 8.0 | | |
| Cetyl Alcohol | 10.0 | 8.0 | 10.0 | 8.0 |
| Liquid Paraffin | 7.5 | | | 6.0 |
| White Vaseline | 10.0 | 12.0 | 10.0 | 15.0 |
| Purified Water | 59.0 | 61.0 | 56.0 | 69.0 |

What we claim is:

1.  A corticosteroid topical preparation which comprises a monoglyceride of a $C_6$-$C_{10}$ medium-chain fatty acid together with the active ingredient.

2.  The preparation claimed in Claim 1, wherein the preparation contains 0.01-2.00 weight percent of a corticosteroid and 0.5-20.0 weight percent of a monoglyceride of a $C_6$-$C_{10}$ medium-chain fatty acid.

3.  The preparation claimed in Claim 2, wherein one or more of the following additives/ are contained in addition to the corticosteroid and the monoglyceride:

a.  1-15 wt. percent of a primary, secondary or tertiary $C_2$-$C_6$ alcohol;

b.  1-10 wt. percent of a polyethylene glycol having a mean molecular weight of 200 to 4000;

c.  1-90 wt. percent of an oily substance;

d.  0.5-15 wt. percent of a non-ionic surfactant; and

e.  water.

4.  The preparation claimed in Claim 1, wherein the preparation contains 0.01-2.00 weight percent of a corticosteroid and 1-15 weight percent of a monoglyceride of a $C_6$-$C_{10}$ medium-chain fatty acid.

5.  The preparation claimed in Claim 3, wherein the preparation contains 0.5-5 wt. percent of a surfactant and an oily substance.

6.   The preparation claimed in Claim 1, wherein the preparation contains 0.5-15 wt. percent of a surfactant, 2-35 wt. percent of an oily substance and water.

7.   The preparation claimed in Claim 3, wherein the preparation contains 1-15 wt. percent of an alcohol, 0.5-15 wt. percent of a surfactant, 2-35 wt. percent of an oily substance and water.

8.   The preparation claimed in Claim 3, wherein the preparation contains 1-10 wt. percent of a polyethylene glycol, 0.5-15 wt. percent of a surfactant, 2-35 wt. percent of an oily substance and water.

9.   The preparation claimed in Claim 4, wherein the preparation contains 1-15 wt. percent of an alcohol, 0.5-5 wt. percent of a surfactant and an oily substance.

10.   The preparation claimed in Claim 4, wherein the preparation contains 1-10 wt. percent of a polyethylene glycol, 0.5-5 wt. percent of a surfactant and an oily substance.

11.   The preparation claimed in Claim 4, wherein the preparation contains 1-10 wt. percent of an alcohol and a polyethylene glycol, 0.5-5 wt. percent of a surfactant and an oily substance.

12.   The preparation claimed in Claim 4, wherein a solubilizing agent is contained in addition to the corticosteroid and the monoglyceride.

13.   The preparation claimed in Claim 1, wherein the corticosteroid is selected from the group consisting of betamethasone valerate, betamethasone dipropionate, alclometasone dipropionate, dexamethasone, triamcinolone acetonide, fluocinonide and fluocinolone acetonide.

14. The preparation claimed in Claim 1, wherein the corticosteroid is selected from the group consisting of betamethasone valerate, betamethasone dipropionate and alclometasone dipropionate.

15. The preparation claimed in Claim 1, wherein the monoglyceride is selected from the group consisting of glycerol monohexanoate, glycerol monooctanoate and glycerol monodecanoate.

16. A process for the production of a corticosteroid topical preparation with an improved activity which comprises/ adding a monoglyceride of a $C_6$-$C_{10}$ medium-chain fatty acid together with the active ingredient to the topical preparation.